# EUROPEAN PATENT APPLICATION

(11) **EP 4 741 001 A1**
(43) Date of publication of application: **13.05.2026**
(21) Application number: 25214227.8
(22) Date of filing: 07.11.2025
(51) Int. Cl.: A61N 1/39

(54) **INTELLIGENT DEFIBRILLATOR OPERATING SYSTEM THROUGH MOTION DETECTION**

(30) Priority: 08.11.2024 KR 20240158107; 12.08.2025 KR 20250111511
(71) Applicant: CU Medical Systems Inc., Wonju-si, Gangwon-do 26365 (KR)
(72) Inventor: NOH, Tae Jong, 26439 Wonju-si (KR)
(74) Representative: Schiweck Weinzierl Koch Patentanwälte Partnerschaft mbB

(57) **Abstract**

An intelligent defibrillator operating system through movement detection according to an exemplary embodiment of the present disclosure may include a power supply unit for supplying power to an automated external defibrillator (AED); a display unit provided with a display configured to output a warning notification to guide a user of the AED regarding a usage method of the AED, a defibrillation procedure using the AED, and a necessity of an electric shock based on a defibrillation pulse to a patient; and a voice output unit provided with a speaker; an electrode attached to a part of the patient's body to receive an ECG (electrocardiogram) signal of the patient; a defibrillation pulse unit configured to receive the ECG signal of the patient through the electrode; a sensing unit configured to detect a tilt variation (Δθ), a tilt holding time, an acceleration variation (Δa), a tilt value (0), and holding times of the tilt and acceleration of the AED; and a control unit configured to analyze the ECG signal of the patient received by the defibrillation pulse unit, and to control an operation of the AED based on the tilt, the acceleration, and the holding times of variations in the tilt and the acceleration of the AED.

## Description

### CROSS-REFERENCE TO RELATED APPLICATIONS

This application claims the priority of Korean Patent Application No. 10-2024-0158107 filed on November 8, 2024, in the Korean Intellectual Property Office, the disclosure of which is incorporated herein by reference.

### BACKGROUND

### Field

The present disclosure relates to an intelligent defibrillator operating system and method through motion detection, and more particularly, to an intelligent defibrillator operating system and method through motion detection that is capable of controlling the operation of an automated external defibrillator (AED) based on the tilt, acceleration, and maintenance time of tilt and acceleration changes of the AED.

### Description of the Related Art

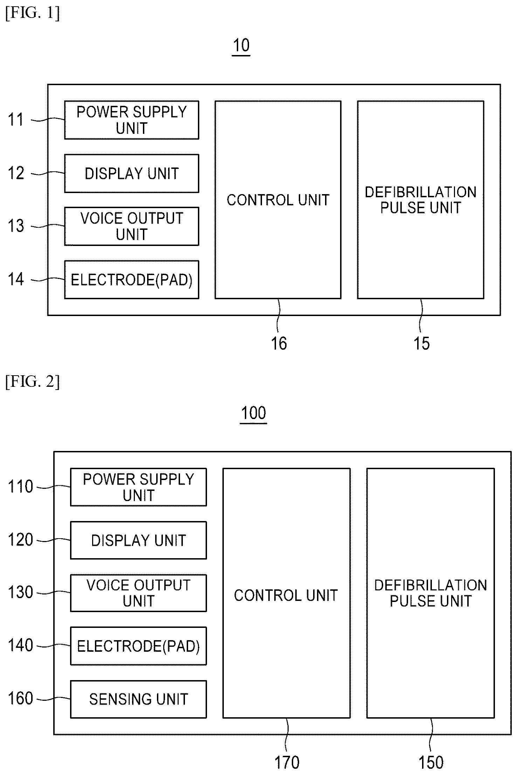

The conventional automated external defibrillator (AED) 10 includes a power supply unit 11, a display unit 12, a voice output unit 13, electrodes 14, a defibrillation pulse unit 15, and a control unit 16, as illustrated in FIG. 1.

The defibrillation pulse unit 15 is connected to the electrodes 14. The electrodes 14 are attached by the user to a part of the patient's body (preferably, the cardiac side), receive the patient's electrocardiogram (ECG) signals, and transmit them to the defibrillation pulse unit 15. The defibrillation pulse unit 15 transmits the ECG signals to the control unit 16.

In addition, when an abnormal pattern (e.g., dangerous cardiac rhythms such as ventricular fibrillation, pulseless ventricular tachycardia, asystole, bradycardia, tachycardia, and others) in the ECG signals is analyzed during the process of analyzing the patient's ECG signals, the control unit 16 controls the display unit 12 or the voice output unit 13 to generate a warning alert and provide the patient's status information to the user.

Further, the control unit 16 controls the defibrillation pulse unit 15 to generate a defibrillation pulse for treating the patient in whom an abnormal pattern in the ECG signals is detected.

Such a conventional automated external defibrillator 10 is typically stored in a designated location. When an emergency situation occurs, and the user carries the automated external defibrillator 10 from the designated location to the patient, then presses a button or opens a cover (lid) by hand, the power supply unit 11 supplies power, thereby converting the automated external defibrillator 10 from an OFF state to an ON state so that a defibrillation process using the automated external defibrillator 10 may proceed.

However, since the conventional automated external defibrillator AED 10 is powered on manually by the user, there is a possibility of operation delay in an emergency situation, thereby causing inconvenience in proceeding with the defibrillation process in the emergency situation.

### [Prior Art Documents]

### [Patent Documents]

(Patent Document 1) Korean Unexamined Patent Publication No. 10-2024-0071505 (published on May 23, 2024)

### SUMMARY

The present disclosure has been devised to solve the above-described problems, and an object of the present disclosure is to provide an intelligent defibrillator operating system and method through motion detection that is capable of controlling the operation of an automated external defibrillator (AED) based on the tilt, acceleration, and maintenance time of tilt and acceleration changes of the AED.

In addition, object of the present disclosure is to provide an intelligent defibrillator operating system and method through motion detection that allows the power of the automated external defibrillator to be automatically supplied according to the movement of the automated external defibrillator, and that may intelligently control the automated external defibrillator so that screen and voice outputs for guiding the use of the automated external defibrillator according to whether the automated external defibrillator is moved and whether the AED reaches a usage position for performing a defibrillation process, as well as a defibrillation process based on defibrillation pulse generation, are automatically performed.

However, the technical objects to be achieved by the present disclosure are not limited to the technical objects mentioned above, and other technical objects not mentioned will be clearly understood by those skilled in the art to which the present disclosure pertains from the following description.

In order to achieve the above object, in an intelligent defibrillator operating system for controlling operation of an automated external defibrillator, an intelligent defibrillator operating system through movement detection according to an exemplary embodiment of the present disclosure may include a power supply unit for supplying power to an automated external defibrillator (AED); a display unit provided with a display configured to output a warning notification to guide a user of the AED regarding a usage method of the AED, a defibrillation procedure using the AED, and a necessity of an electric shock based on a defibrillation pulse to a patient; and a voice output unit provided with a speaker; an electrode attached to a part of the patient's body to receive an ECG (electrocardiogram) signal of the patient; a defibrillation pulse unit configured to receive the ECG signal of the patient through the electrode; a sensing unit configured to detect a tilt variation (Δθ), a tilt holding time, an acceleration variation (Δa), a tilt value (θ), and holding times of the tilt and acceleration of the AED; and a control unit configured to analyze the ECG signal of the patient received by the defibrillation pulse unit, and to control an operation of the AED based on the tilt, the acceleration, and the holding times of variations in the tilt and the acceleration of the AED.

In addition, the defibrillation pulse unit may generate a defibrillation pulse for delivering an electric shock to the patient when the control unit analyzes an abnormal pattern within the ECG signal of the patient, the electrode may provide the electric shock according to the defibrillation pulse to the patient, and the display unit and the voice output unit may output information on patient condition and information on a method of performing a follow-up procedure (CPR) to be carried out after the defibrillation procedure.

The abnormal pattern in the ECG signal may be an ECG signal matched with rhythm types of ventricular fibrillation, pulseless ventricular tachycardia, asystole, bradycardia, and tachycardia.

The patient condition information may include a type of cardiac rhythm of the patient for determining whether the patient is a defibrillation target or a non-defibrillation target, a defibrillation readiness state of the patient by the AED, whether movement of the patient is detected, and whether an electric shock based on a defibrillation pulse is required.

The control unit may control the power supply unit such that power is supplied to the AED based on a tilt variation (Δθ), a tilt holding time, and an acceleration variation (Δa) of the AED.

The control unit may control the display unit and the voice output unit such that, while the power of the AED is supplied and remains in an ON state, the AED is maintained in a standby state before proceeding with a defibrillation procedure based on the acceleration variation (Δa) and the tilt variation (Δθ).

The display unit and the voice output unit may not output a screen and a voice for guiding the user in a usage method of the AED when the AED is in the standby state.

The control unit may determine whether the AED has reached a usage position for proceeding with a defibrillation procedure by the user based on a tilt value (θ), an acceleration variation (Δa), and holding times of the tilt and acceleration (stop holding time) of the AED.

The control unit may control the display unit and the voice output unit such that a defibrillation procedure using the AED is carried out when it is determined that the AED has reached the usage position.

The display unit may output the defibrillation procedure using the AED to the display for guiding the user, and the voice output unit may output a voice through the speaker for guiding the usage method of the AED.

The present disclosure enables a defibrillation process to be carried out in an emergency situation without operation delay of an automated external defibrillator (AED), since the power of the AED is automatically supplied according to the movement of the AED.

In addition, the present disclosure may provide convenience in the defibrillation process, since the screen and voice outputs for guiding the use of the AED according to whether the AED is moved and whether the AED reaches a usage position for performing the defibrillation process, as well as the defibrillation process based on defibrillation pulse generation, are automatically performed.

Meanwhile, the effects obtainable from the present disclosure are not limited to the effects mentioned above, and other effects not mentioned will be clearly understood by those skilled in the art to which the present disclosure pertains from the following description.

The effects of the present disclosure are not limited to the aforementioned effects, and other effects, which are not mentioned above, will be apparently understood to a person having ordinary skill in the art from the following description.

The objects to be achieved by the present disclosure, the means for achieving the objects, and the effects of the present disclosure described above do not specify essential features of the claims, and, thus, the scope of the claims is not limited to the disclosure of the present disclosure.

### BRIEF DESCRIPTION OF DRAWINGS

The above and other aspects, features and other advantages of the present disclosure will be more clearly understood from the following detailed description taken in conjunction with the accompanying drawings, in which:
FIG. 1 is an exemplary view illustrating a conventional automated external defibrillator (AED);
FIG. 2 is an exemplary view illustrating an intelligent defibrillator operating system through movement detection according to an exemplary embodiment of the present disclosure;
FIG. 3 is a flowchart illustrating a power supply method according to an exemplary embodiment of the present disclosure;
FIG. 4 is a flowchart illustrating a first standby operation and defibrillator operating method according to an exemplary embodiment of the present disclosure;
FIG. 5 is a flowchart illustrating a second standby operation and defibrillator operating method according to an exemplary embodiment of the present disclosure; and
FIG. 6 is a flowchart illustrating a defibrillation procedure progress method according to an exemplary embodiment of the present disclosure.

### DETAILED DESCRIPTION OF THE EMBODIMENT

Hereinafter, the exemplary embodiment of the present disclosure will be described with reference to the accompanying drawings and exemplary embodiments as follows. Scales of components illustrated in the accompanying drawings are different from the real scales for the purpose of description, so that the scales are not limited to those illustrated in the drawings.

Hereinafter, exemplary embodiments of the present disclosure will be described in detail with reference to the accompanying drawings so that those skilled in the art to which the present disclosure pertains may readily implement the present disclosure. However, the description of the present disclosure is merely exemplary for structural and functional explanation, and the scope of the present disclosure shall not be construed as being limited to the embodiments described herein. That is, the embodiments may be modified in various ways and may take various forms, and the scope of the present disclosure shall be understood to include equivalents capable of implementing the technical spirit. In addition, objects or effects presented in the present disclosure do not mean that a specific embodiment must include all of them or only such effects, and the scope of the present disclosure shall not be construed as being limited thereto.

The meanings of terms described in the present disclosure should be understood as follows.

The terms "first," "second," and the like are used to distinguish one component from another, and the scope of rights shall not be limited by these terms. For example, a first component may be referred to as a second component, and similarly, a second component may also be referred to as a first component. When a component is described as being "connected to" another component, it should be understood that the component may be directly connected to the other component, or that another component may be interposed therebetween. On the other hand, when a component is described as being "directly connected to" another component, it should be understood that no other component is interposed therebetween. Likewise, other expressions describing relationships between components, such as "between ... and ..." versus "directly between ... and ..." or "adjacent to" versus "directly adjacent to," should be interpreted in the same manner.

Expressions in the singular form shall be understood to include the plural form unless clearly indicated otherwise in the context, and terms such as "comprise" or "have" are intended to specify that the described features, numbers, steps, operations, components, parts, or combinations thereof exist, and shall not preclude the possibility of the existence or addition of one or more other features, numbers, steps, operations, components, parts, or combinations thereof.

All terms used herein shall be interpreted as having meanings commonly understood by those skilled in the art to which the present disclosure pertains unless otherwise defined. Terms defined in generally used dictionaries shall be interpreted as having meanings consistent with their contextual meanings in the related art, and unless explicitly defined in the present disclosure, shall not be interpreted as having idealized or excessively formal meanings.

### Intelligent Defibrillator Operating System through Movement Detection

Hereinafter, an intelligent defibrillator operating system through movement detection 100 according to an exemplary embodiment of the present disclosure will be described in detail with reference to the accompanying drawings.

FIG. 2 is an exemplary view illustrating an intelligent defibrillator operating system through movement detection according to an exemplary embodiment of the present disclosure.

Referring to FIG. 2, an intelligent defibrillator operating system 100 according to an exemplary embodiment of the present disclosure is configured in an automated external defibrillator (AED) and includes a power supply unit 110, a display unit 120, a voice output unit 130, an electrode 140, a defibrillation pulse unit 150, a sensing unit 160, and a control unit 170.

In one exemplary embodiment, when an emergency occurs and movement begins from a predetermined location (e.g., a storage compartment in a subway station) by a user, the power supply unit 110 may supply power to the AED and switch the AED from an OFF state to an ON state according to information detected in real time by the sensing unit 160.

That is, the power supply unit 110 may automatically supply power to the AED according to information detected in real time by the sensing unit 160, rather than by a user manually supplying power to the AED.

In one exemplary embodiment, the display unit 120 and the voice output unit 130 may output information such as a usage method of the AED, a defibrillation procedure using the AED, a warning notification for guiding a user of the AED regarding a necessity of an electric shock based on a defibrillation pulse to a patient, patient condition information, and a method of performing follow-up procedure (CPR) to be carried out after the defibrillation procedure.

In one exemplary embodiment, the display unit 120 may include a display (not shown). When the control unit 170 analyzes an abnormal pattern in the ECG signal (e.g., an ECG signal matched with dangerous cardiac rhythm types such as ventricular fibrillation, pulseless ventricular tachycardia, asystole, bradycardia, and tachycardia) during analysis of an ECG (electrocardiogram) signal of the patient, the display unit may guide, through the display, patient condition information and a method of performing follow-up procedure (CPR) to be carried out after the defibrillation procedure, and the display may output information regarding patient condition and the follow-up procedure (CPR).

Here, the patient condition information includes a type of cardiac rhythm of the patient (whether the patient is a defibrillation target or a non-defibrillation target), a defibrillation readiness state of the patient (in charging or ready for electric shock), whether movement of the patient is detected, and whether an electric shock based on a defibrillation pulse is required.

In one exemplary embodiment, the voice output unit 130 may include a speaker (not shown). When the control unit 170 analyzes an abnormal pattern in the ECG signal in a process of analyzing an ECG signal of the patient, the voice output unit 130 may guide, through the speaker, a user regarding a necessity of an electric shock based on a defibrillation pulse to a patient, and the speaker may output a warning notification informing the user that an electric shock based on a defibrillation pulse is required for the patient.

In one exemplary embodiment, the electrode 140 is connected to the defibrillation pulse unit 150. When the electrode 140 is attached to a part of the patient's body (preferably near the heart) while power from the power supply unit 110 is supplied to the defibrillation pulse unit 150, the electrode 140 may receive the patient's ECG signal and transmit it to the defibrillation pulse unit 150.

In addition, the electrode 140 may provide an electric shock to the patient according to the defibrillation pulse generated by the defibrillation pulse unit 150 so that a defibrillation procedure using the AED is carried out.

In one exemplary embodiment, the defibrillation pulse unit 150 is connected to the electrode 140 and the control unit 170, and the electrode 140 in contact with a part of the patient's body may transmit an ECG (electrocardiogram) signal of the patient measured thereby to the control unit 170.

In addition, the defibrillation pulse unit 150 may also generate a defibrillation pulse for delivering an electric shock to the patient when the control unit 170 analyzes an abnormal pattern in the ECG signal in a process of analyzing an ECG signal of the patient.

In one exemplary embodiment, the sensing unit 160 includes a tilt sensor, a gyro sensor, and a three-axis accelerometer, and may detect in real time a tilt variation (Δθ), a tilt holding time, an acceleration variation (Δa), a tilt value (θ), and holding times of the tilt and acceleration (stop holding time) of the AED.

In one exemplary embodiment, the control unit 170 may control the power supply unit 110 to supply power to the AED based on the tilt variation (Δθ), the tilt holding time, and the acceleration variation (Δa) of the AED detected in real time by the sensing unit 160.

The control unit 170 may also control the display unit 120 and the voice output unit 130 such that, while power is supplied to the AED and the power of the AED remains in an ON state, the AED is maintained in a standby state before proceeding with a defibrillation procedure based on the acceleration variation (Δa) and the tilt variation (Δθ) detected in real time by the sensing unit 160.

In this case, the display unit 120 and the voice output unit 130 may not output a screen and a voice for guiding the user in a usage method of the AED when the AED is in the standby state.

Further, the control unit 170 may determine whether the AED has reached a usage position (beside the patient) for proceeding with a defibrillation procedure by the user based on the tilt value (θ), the acceleration variation (Δa), and the holding times of the tilt and acceleration (stop holding time) of the AED detected in real time by the sensing unit 160.

The control unit 170 may also control the display unit 120 and the voice output unit 130 such that a defibrillation procedure using the AED is carried out when it is determined that the AED has reached the usage position.

At this time, the display unit 120 and the voice output unit 130 may output a usage method of the AED and a defibrillation procedure using the AED for guiding the user.

Furthermore, when the control unit 170 analyzes an abnormal pattern in the ECG signal of the patient in a process of analyzing an ECG (electrocardiogram) signal of the patient, the control unit 170 may control the display unit 120 and the voice output unit 130 to guide the user regarding patient condition information and a method of performing follow-up procedure (CPR) to be carried out after the defibrillation procedure.

At this time, the display unit 120 and the voice output unit 130 may output information regarding patient condition and the follow-up procedure (CPR) to be carried out after the defibrillation procedure.

Moreover, even when physical conditions such as the tilt value (θ), the acceleration variation (Δa), and the holding times of the tilt and acceleration (stop holding time) of the AED correspond to operation conditions for the AED to proceed with a defibrillation procedure, the display unit 120 may be provided, on one side of the display, with an operation means (not shown) for forcibly operating the AED in case the AED remains in the standby state due to a recognition (determination) error of the control unit 170.

### Intelligent Defibrillator Operating Method through Movement Detection

Hereinafter, an intelligent defibrillator operating method through movement detection according to an exemplary embodiment of the present disclosure, which is performed by the intelligent defibrillator operating system 100 described above, will be described in detail with reference to the accompanying drawings.

### 1) Power Supply Method

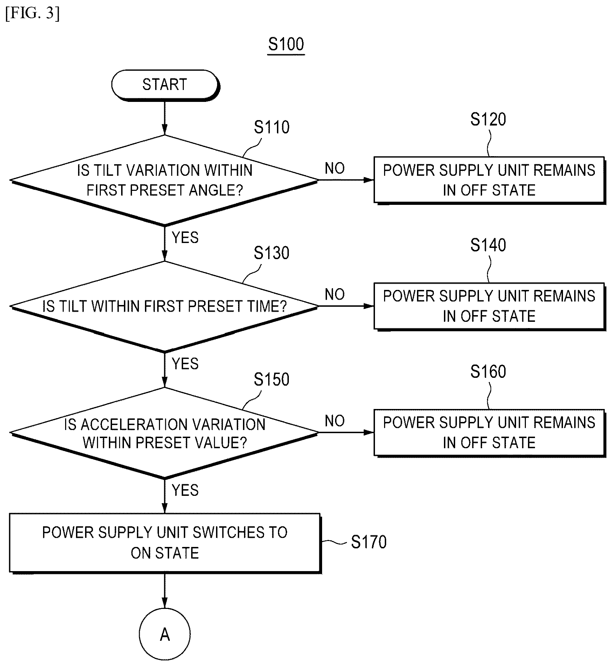

FIG. 3 is a flowchart illustrating a power supply method according to an exemplary embodiment of the present disclosure.

Referring to FIG. 3, the control unit 170 may determine whether physical movement of the AED occurs, and may control the power supply unit 110 such that power from the power supply unit 110 is supplied to the AED when preset conditions are satisfied.

First, in order to detect whether physical movement of the AED occurs from a predetermined storage location by a user, the control unit 170 may determine whether a tilt variation (Δθ) of the AED detected in real time by the sensing unit 160 is within a first preset angle (S110).

In one exemplary embodiment, the first preset angle may be in a range of 1 to 20°, and preferably 5° or more.

When the tilt variation (Δθ) of the AED is equal to or less than the first preset angle (e.g., 5°) (S110-NO), the control unit 170 may determine that no physical movement of the AED has occurred, and may control the power supply unit 110 such that no power is supplied to the AED, so that the AED remains in an OFF state (S120).

On the other hand, when the tilt variation (Δθ) of the AED is equal to or greater than the first preset angle (e.g., 5°) (S110-YES), in order to detect whether continuous movement occurs in the AED, the control unit 170 may determine whether a tilt holding time of the AED detected in real time by the sensing unit 160 is within a first preset time (S130).

In one exemplary embodiment, the first preset time may be in a range of 100 milliseconds (ms) to 1 second (sec), and preferably 500 ms or more.

At this time, when the tilt holding time of the AED is equal to or less than the first preset time (e.g., 500 ms) (S130-NO), the control unit 170 may determine that only a temporary movement of the AED occurs, and may control the power supply unit 110 such that no power is supplied to the AED, so that the AED remains in an OFF state (S140).

On the other hand, when the tilt holding time of the AED is equal to or greater than the first preset time (e.g., 500 ms) (S130-YES), in order to detect physical movement or vibration of the AED, the control unit 170 may determine whether an acceleration variation (Δa) of the AED detected in real time by the sensing unit 160 is within a preset value (in units of g) (S150).

In one exemplary embodiment, the preset value may be in a range of 0.1 g to 1.5 g (gravitational acceleration), and preferably 0.1 g or more.

At this time, when the acceleration variation (Δa) of the AED is equal to or less than the preset value (e.g., 0.1 g) (S150-NO), the control unit 170 may determine that no physical movement or vibration of the AED is detected, and may control the power supply unit 110 such that no power from the power supply unit 110 is supplied to the AED, so that the AED remains in an OFF state (S160).

On the other hand, when the acceleration variation (Δa) of the AED is equal to or greater than the preset value (e.g., 0.1 g) (S150-YES), the control unit 170 may control the power supply unit 110 such that power from the power supply unit 110 is supplied to the AED, so that the AED may be switched to an ON state (S170).

That is, in one exemplary embodiment, when the tilt variation (Δθ) of the AED detected in real time by the sensing unit 160 is equal to or greater than the first preset angle, the tilt holding time of the AED is equal to or greater than the first preset time, and additionally the acceleration variation (Δa) of the AED is equal to or greater than the preset value, the control unit 170 may control the power supply unit 110 to supply power to the AED from the power supply unit 110.

### 2) First Standby Operation and Defibrillator Operating Method

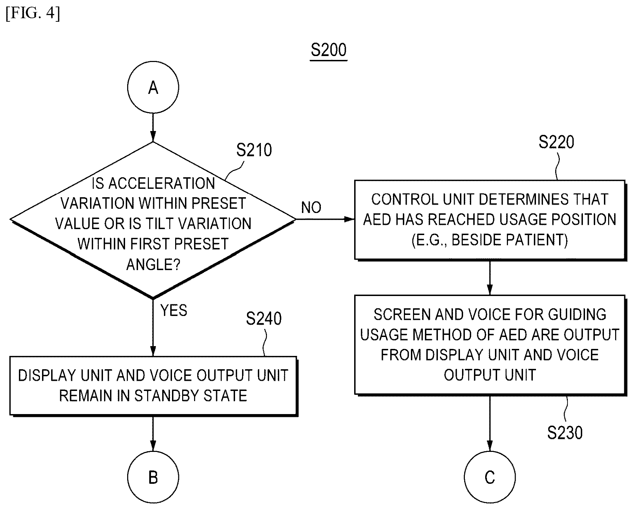

FIG. 4 is a flowchart illustrating a first standby operation and defibrillator operating method according to an exemplary embodiment of the present disclosure.

Referring to FIG. 4, the control unit 170 may determine whether continuous movement occurs in the AED supplied with power according to preset conditions of step S210 to be described below, and may control the display unit 120 and the voice output unit 130 such that a defibrillation procedure in the AED is either carried out or not carried out.

First, in order to detect whether continuous movement of the AED supplied with power occurs, the control unit 170 may determine whether an acceleration variation (Δa) of the AED supplied with power is equal to or less than a preset value (e.g., 0.1 g), or whether a tilt variation (Δθ) of the AED is equal to or less than a first preset angle (e.g., 5°°) (S210).

At this time, when the acceleration variation (Δa) of the AED supplied with power is equal to or less than the preset value (e.g., 0.1 g) or the tilt variation (Δθ) is equal to or less than the first preset angle (e.g., 5°°) (S210-YES), the control unit 170 may determine that continuous movement does not occur in the AED, and that the AED has reached a usage position (beside the patient) for AED operation (S220).

Thereafter, in order to guide the user in a usage method of the AED, the control unit 170 may control the display unit 120 and the voice output unit 130 such that a screen and a voice for guiding the usage method of the AED are output from the display unit 120 and the voice output unit 130 (S230).

On the other hand, when the acceleration variation (Δa) of the AED supplied with power is equal to or greater than the preset value (e.g., 0.1 g) or the tilt variation (Δθ) is equal to or greater than the first preset angle (e.g., 5°) (S210-NO), the control unit 170 may determine that the AED has not reached the usage position (beside the patient) for proceeding with a defibrillation procedure, and that continuous movement occurs in the AED. In this case, the control unit 170 may maintain the AED in the ON state, but may control the display unit 120 and the voice output unit 130 such that a screen and a voice for guiding the usage method of the AED are not output (S240).

That is, in one exemplary embodiment, when the acceleration variation (Δa) of the AED supplied with power is equal to or less than the preset value (e.g., 0.1 g) or the tilt variation (Δθ) is equal to or less than the first preset angle (e.g., 5°), the control unit 170 may determine that no movement of the AED occurs, and may control the display unit 120 and the voice output unit 130 such that a defibrillation procedure is carried out.

### 3) Second Standby Operation and Defibrillator Operating Method

FIG. 4 is a flowchart illustrating a second standby operation and defibrillator operating method according to an exemplary embodiment of the present disclosure.

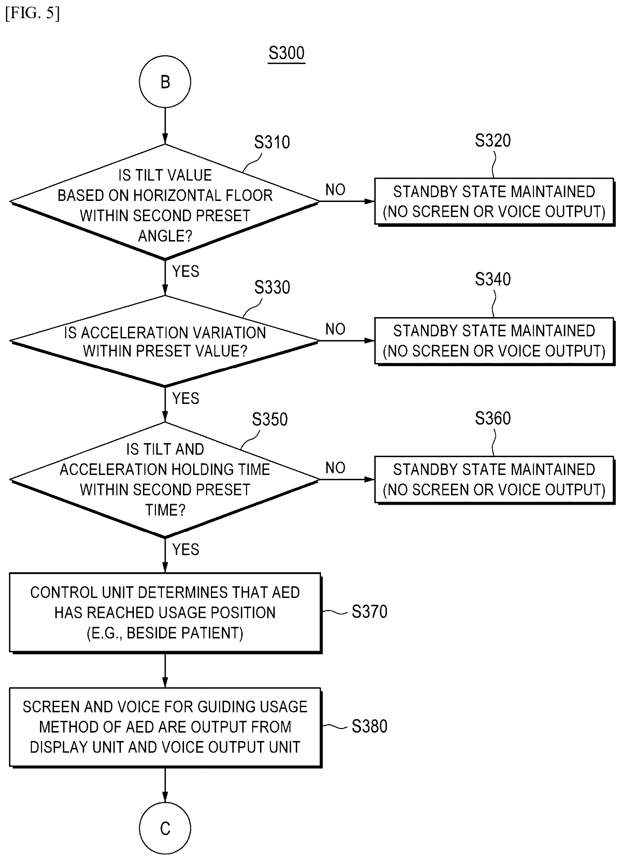

Referring to FIG. 5, after the standby state maintaining step (S240) of FIG. 4, the control unit 170 may additionally determine whether continuous movement occurs in the AED supplied with power according to preset conditions of steps S310, S330, and S350 to be described below, and may control the display unit 120 and the voice output unit 130 such that a defibrillation procedure in the AED is either carried out or not carried out.

First, in order to detect whether continuous movement of the AED occurs, the control unit 170 may determine whether a tilt value (θ) of the AED supplied with power, based on a horizontal floor, is within a second preset angle (S310).

In step S310, the second preset angle may be in a range of 0 to 45° based on a horizontal floor, and preferably 15°° based on a horizontal floor.

At this time, when the tilt value (θ) of the AED supplied with power, based on a horizontal floor, is equal to or greater than the second preset angle (e.g., 15° based on a horizontal floor) (S310-NO), the control unit 170 may determine that the AED has not reached a usage position (beside the patient) for proceeding with a defibrillation procedure, and that continuous movement occurs in the AED. In this case, the control unit 170 may maintain the AED in the ON state, but may control the display unit 120 and the voice output unit 130 such that a screen and a voice for guiding a usage method of the AED are not output (S320).

On the other hand, when the tilt value (θ) of the AED supplied with power, based on a horizontal floor, is equal to or less than the second preset angle (e.g., 15° based on a horizontal floor) (S310-YES), the control unit 170 may determine whether an acceleration variation (Δa) of the AED supplied with power is equal to or greater than a preset value (e.g., 0.1 g) in order to detect whether continuous movement occurs in the AED (S330).

At this time, when the acceleration variation (Δa) of the AED supplied with power is equal to or less than the preset value (e.g., 0.1 g) (S330-NO), the control unit 170 may determine that continuous movement does not occur in the AED, and may maintain the AED in an ON state, but may control the display unit 120 and the voice output unit 130 such that a screen and a voice for guiding a usage method of the AED are not output from the display unit 120 and the voice output unit 130 (S340).

On the other hand, when the acceleration variation (Δa) of the AED supplied with power is equal to or greater than the preset value (e.g., 0.1 g) (S330-YES), the control unit 170 may determine whether a state in which the tilt value (θ) of the AED supplied with power is equal to or greater than the second preset angle and the acceleration variation (Δa) is equal to or greater than the preset value is maintained for a time equal to or greater than the second preset time, in order to detect whether continuous movement occurs in the AED (S350).

In step S350, the second preset time may be in a range of 500 ms to 2 sec, and preferably 1 sec.

When the state in which the tilt value (θ) of the AED supplied with power is equal to or greater than the second preset angle and the acceleration variation (Δa) is equal to or greater than the preset value is maintained for a time equal to or greater than the second preset time (e.g., 1 sec) (S350-NO), the control unit 170 may determine that the AED has not reached the usage position (beside the patient) for proceeding with a defibrillation procedure, and that continuous movement occurs in the AED. In this case, the control unit 170 may maintain the AED in the ON state, but may control the display unit 120 and the voice output unit 130 such that a screen and a voice for guiding a usage method of the AED are not output (S360).

On the other hand, when the state in which the tilt value (θ) of the AED supplied with power is equal to or less than the second preset angle and the acceleration variation (Δa) is equal to or greater than the preset value is maintained for a time equal to or less than the second preset time (e.g., 1 sec) (S350-YES), the control unit 170 may determine that continuous movement does not occur in the AED, and that the AED has reached a usage position (beside the patient) for AED operation (S370).

Thereafter, in order to guide the user in a usage method of the AED, the control unit 170 may control the display unit 120 and the voice output unit 130 such that a screen and a voice for guiding the usage method of the AED are output (S380).

That is, in one exemplary embodiment, after the standby state maintaining step (S240) of FIG. 4, when the tilt value (θ) of the AED supplied with power based on a horizontal floor is equal to or less than the second preset angle (e.g., 15° based on a horizontal floor), the acceleration variation (Δa) of the AED supplied with power is equal to or less than the preset value (e.g., 0.1 g), and simultaneously the tilt value (θ) is equal to or less than the second preset angle range while the state in which the acceleration variation (Δa) is equal to or greater than the preset value is maintained for a time equal to or less than the second preset time (e.g., 1 sec), the control unit 170 may determine that no movement of the AED occurs, and may control the display unit 120 and the voice output unit 130 such that a defibrillation procedure is carried out.

### 4) Defibrillation Procedure Progress Method

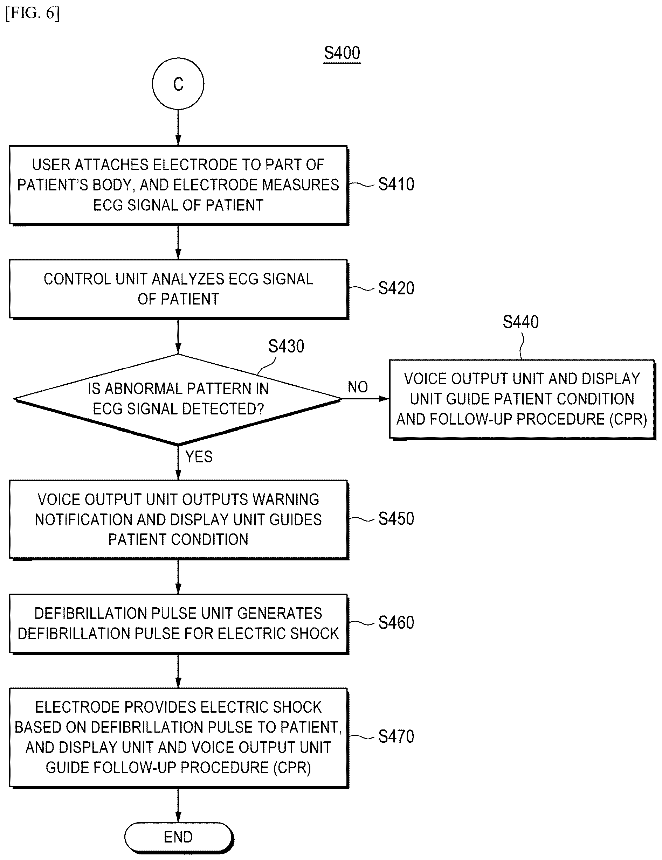

FIG. 6 is a flowchart illustrating a defibrillation procedure progress method according to an exemplary embodiment of the present disclosure.

Referring to FIG. 6, when a screen and a voice for guiding a usage method of the AED are output from the display unit 120 and the voice output unit 130, a user may attach the electrode 140 to a part of a patient's body (near the heart) with reference to the screen and the voice, and the electrode 140 attached to the patient's body may receive an ECG (electrocardiogram) signal of the patient (S410).

Thereafter, the control unit 170 may analyze the ECG signal of the patient received through the defibrillation pulse unit 150 by the electrode 140 (S420), and may detect an abnormal pattern (e.g., an ECG signal matched with dangerous cardiac rhythm types such as ventricular fibrillation, pulseless ventricular tachycardia, asystole, bradycardia, and tachycardia) in the ECG signal of the patient (S430).

At this time, when an abnormal pattern (e.g., an ECG signal matched with dangerous cardiac rhythm types such as ventricular fibrillation, pulseless ventricular tachycardia, asystole, bradycardia, and tachycardia) is not detected from the ECG signal of the patient (S430-NO), the control unit 170 may control the display unit 120 and the voice output unit 130 such that information regarding patient condition and a follow-up procedure (CPR) is output from the display of the display unit 120 and the speaker of the voice output unit 130 in order to guide the user (S440).

In step S440, among the patient condition information output from the display unit 120 and the voice output unit 130, a cardiac rhythm type of the patient may be a cardiac rhythm of a non-defibrillation target, and whether an electric shock based on a defibrillation pulse is required may preferably indicate that the electric shock is unnecessary.

On the other hand, when an abnormal pattern (e.g., an ECG signal matched with dangerous cardiac rhythm types such as ventricular fibrillation, pulseless ventricular tachycardia, asystole, bradycardia, and tachycardia) is detected from the ECG signal of the patient (S430-YES), the control unit 170 may control the display unit 120 and the voice output unit 130 such that a warning notification for guiding the user that an electric shock based on a defibrillation pulse is required is output from the voice output unit 130, in order to guide the user that the electric shock based on the defibrillation pulse is required for the patient. In addition, the control unit 170 may control the display unit 120 such that patient condition information is output from the display of the display unit 120 in order to guide the user regarding patient condition information (S450).

In step S440 among the patient condition information output from the display of the display unit 120, a cardiac rhythm type of the patient may be a cardiac rhythm of a defibrillation target, and in the case of the cardiac rhythm of the defibrillation target, it is preferable that an electric shock based on a defibrillation pulse is required.

Thereafter, the defibrillation pulse unit 150 may generate a defibrillation pulse for delivering an electric shock in order to perform a defibrillation procedure using the AED (S460).

Subsequently, the electrode 140 attached to a part of the patient's body may provide the electric shock based on the defibrillation pulse generated by the defibrillation pulse unit 150 to the patient so that the defibrillation procedure is carried out, and the control unit 170 may control the display unit 120 and the voice output unit 130 such that information regarding patient condition and a follow-up procedure (CPR) is output in order to guide the user (S470).

Thereafter, the user may perform a follow-up procedure (CPR) according to a method of performing the follow-up procedure output from the display unit 120 and the voice output unit 130, thereby responding to the patient's emergency situation.

### Effects of the Present Disclosure

The intelligent defibrillator operating system 100 according to the present disclosure may proceed with a defibrillation procedure in an emergency situation without delay in operation of the AED, since power of the AED is automatically supplied according to movement of the AED.

In addition, the intelligent defibrillator operating system 100 according to the present disclosure may provide convenience in a defibrillation procedure, since screen and voice outputs for guiding a usage of the AED are performed according to whether the AED has moved and whether the AED has reached a usage position for proceeding with the defibrillation procedure, and since the defibrillation procedure based on generation of a defibrillation pulse is automatically carried out.

As described above, the detailed description of the preferred embodiments disclosed herein has been provided to enable those skilled in the art to implement and practice the present disclosure. Although the preferred embodiments of the present disclosure have been described with reference to the above, it will be understood by those skilled in the art that various modifications and changes can be made to the present disclosure without departing from the scope of the present disclosure. For example, those skilled in the art may utilize each of the configurations described in the above embodiments in combination. Therefore, the present disclosure is not intended to be limited to the embodiments shown herein, but is to be accorded the broadest scope consistent with the principles and novel features disclosed herein.

The present disclosure may be embodied in other specific forms without departing from the technical spirit and essential features of the present disclosure. Therefore, the above detailed description should not be construed as restrictive in any respect but should be considered illustrative. The scope of the present disclosure should be determined by reasonable interpretation of the appended claims, and all modifications within the equivalent scope of the present disclosure are included within the scope of the present disclosure. The present disclosure is not intended to be limited to the embodiments shown herein, but is to be accorded the broadest scope consistent with the principles and novel features disclosed herein. In addition, claims without an explicit reference relationship in the claims may be combined to constitute an embodiment, or may be included as new claims by amendment after filing.

## Claims

1. An intelligent defibrillator operating system through movement detection, the intelligent defibrillator operating system for controlling an operation of an automated external defibrillator (AED), comprising:
a power supply unit for supplying power to the AED;
a display unit provided with a display configured to output a warning notification to guide a user of the AED regarding a usage method of the AED, a defibrillation procedure using the AED, and a necessity of an electric shock based on a defibrillation pulse to a patient; and a voice output unit provided with a speaker;
an electrode attached to a part of the patient's body to receive an ECG (electrocardiogram) signal of the patient;
a defibrillation pulse unit configured to receive the ECG signal of the patient through the electrode;
a sensing unit configured to detect a tilt variation (Δθ), a tilt holding time, an acceleration variation (Δa), a tilt value (θ), and holding times of the tilt and acceleration of the AED; and
a control unit configured to analyze the ECG signal of the patient received by the defibrillation pulse unit, and to control the operation of the AED based on the tilt, the acceleration, and the holding times of variations in the tilt and the acceleration of the AED.

2. The intelligent defibrillator operating system according to claim 1, wherein the defibrillation pulse unit generates a defibrillation pulse for delivering an electric shock to the patient when the control unit analyzes an abnormal pattem within the ECG signal of the patient,
the electrode provides the electric shock according to the defibrillation pulse to the patient, and
the display unit and the voice output unit output information on patient condition and information on a method of performing a follow-up procedure (CPR) to be carried out after the defibrillation procedure.

3. The intelligent defibrillator operating system according to claim 2, wherein the abnormal pattern in the ECG signal is an ECG signal matched with rhythm types of ventricular fibrillation, pulseless ventricular tachycardia, asystole, bradycardia, and tachycardia.

4. The intelligent defibrillator operating system according to claim 2, wherein the patient condition information includes a type of cardiac rhythm of the patient for determining whether the patient is a defibrillation target or a non-defibrillation target, a defibrillation readiness state of the patient by the AED, whether movement of the patient is detected, and whether an electric shock based on the defibrillation pulse is required.

5. The intelligent defibrillator operating system according to claim 1, wherein the control unit controls the power supply unit such that power is supplied to the AED based on the tilt variation (Δθ), the tilt holding time, and the acceleration variation (Δa) of the AED.

6. The intelligent defibrillator operating system according to claim 5, wherein the control unit controls the display unit and the voice output unit such that, while the power of the AED is supplied and remains in an ON state, the AED is maintained in a standby state before proceeding with the defibrillation procedure based on the acceleration variation (Δa) and the tilt variation (Δθ).

7. The intelligent defibrillator operating system according to claim 6, wherein the display unit and the voice output unit do not output a screen and a voice for guiding the user in the usage method of the AED when the AED is in the standby state.

8. The intelligent defibrillator operating system according to claim 6, wherein the control unit determines whether the AED has reached a usage position for proceeding with the defibrillation procedure by the user based on the tilt value (θ), the acceleration variation (Δa), and the holding times of the tilt and acceleration (stop holding time) of the AED.

9. The intelligent defibrillator operating system according to claim 8, wherein the control unit controls the display unit and the voice output unit such that the defibrillation procedure using the AED is carried out when it is determined that the AED has reached the usage position.

10. The intelligent defibrillator operating system according to claim 9, wherein the display unit outputs the defibrillation procedure using the AED to the display for guiding the user, and
the voice output unit outputs a voice through the speaker for guiding the usage method of the AED.
